# FASCICULE DE BREVET EUROPEEN

(11) **EP 3 177 922 B1**
(45) Date de publication et mention de la délivrance du brevet: **09.12.2020**
(21) Numéro de dépôt: 15760211.1
(22) Date de dépôt: 04.08.2015
(51) Int. Cl.: G01N 33/49, G01N 33/68

(54) **PROCÉDÉ POUR LE DÉPISTAGE DE LA DRÉPANOCYTOSE**
VERFAHREN ZUR ERKENNUNG DER SICHELZELLKRANKHEIT
METHOD FOR DETECTING SICKLE-CELL DISEASE

(30) Priorité: 05.08.2014 FR 1457609; 18.12.2014 FR 1462701
(43) Date de publication de la demande: 14.06.2017
(73) Titulaire: Screencell, 95200 Sarcelles (FR)
(72) Inventeur: ZNATY, David, F-75007 Paris (FR); FORESTIER, François, F-75014 Paris (FR); UZAN, Georges, F-94400 Vitry Sur Seine (FR); AUCANT, Cécile, F-25320 Boussieres (FR)
(74) Mandataire: Ipside
(86) Numéro de dépôt international: PCT/FR2015/052148
(87) Numéro de publication internationale: WO 2016/020616

(56) Documents cités:
- WO-A2-2006/026735
- US-A1- 2012 077 218
- By Lennette ET AL: "Cetiedil: Its Potential Usefulness in Sickle Cell Disease", Blood, 1 février 1980 (1980-02-01), XP055175589, Extrait de l'Internet: URL:http://www.bloodjournal.org/content/bl oodjournal/55/2/265.full.pdf [extrait le 2015-03-11]
- Edward J Hicks ET AL: "H b-type Total Percent Comparison of Results for Three Methods of Hemoglobin S Identification Analytical Methods Table 2. Sensitivity and Specificity of Various Screening Procedures for Sickle Cell Dlseasea", CLIN. CHEM. CLINICAL CHEMISTRY, 1 janvier 1973 (1973-01-01), pages 533-535, XP055175588, Extrait de l'Internet: URL:http://www.clinchem.org/content/19/5/5 33.full.pdf [extrait le 2015-03-11]

## Description

La présente invention s'inscrit dans le domaine du dépistage de la drépanocytose. Plus particulièrement, elle concerne un procédé pour un tel dépistage à partir d'un échantillon de sang d'un individu.

La drépanocytose, également dite anémie falciforme, est une maladie génétique autosomale récessive, due à une anomalie de l'hémoglobine contenue dans les globules rouges, plus précisément une anomalie de structure de la chaîne β de l'hémoglobine, caractérisée par la mutation d'un acide aminé de cette chaîne β, de l'acide glutamique en valine.

La drépanocytose constitue l'hémoglobinopathie la plus importante dans le monde. Près de 120 millions d'individus seraient en effet porteurs du trait drépanocytaire.

On retrouve la drépanocytose dans certaines régions du sous-continent indien, en Amérique du Sud et dans le bassin méditerranéen. L'Afrique intertropicale, c'est-à-dire le territoire situé entre le 15^{ème} parallèle sud et le 20^{ème} parallèle nord, est la plus touchée, avec un taux d'environ 1 cas de drépanocytose sur 65 naissances. Les pays les plus atteints sont la République démocratique du Congo, le Sénégal, le Bénin et l'Angola. On peut citer une corrélation, dans ces zones, avec la résistance au paludisme, ce qui conforte l'hypothèse selon laquelle la drépanocytose résulterait d'un processus de sélection naturelle par la résistance au paludisme, due à la difficulté pour le plasmodium d'effectuer son cycle dans les drépanocytes.

En France, la drépanocytose homozygote représente une naissance sur 1200. Il s'agit du premier risque génétique de l'Ile de France. Aux Antilles, on atteint un taux de 1 cas sur 260 naissances. La drépanocytose touche également la population afro-américaine, ce qui s'explique aisément par l'origine historique de la naissance de cette communauté aux Etats-Unis, à savoir la déportation massive d'africains dans le cadre du commerce triangulaire dont les descendants restent aujourd'hui concernés par la drépanocytose.

La drépanocytose constitue ainsi un véritable problème de santé publique, et ce d'autant plus que les porteurs sains, ne manifestant aucun signe clinique, peuvent ignorer qu'ils sont capables de transmettre cette maladie à leur descendance. L'Organisation Mondiale de la Santé prévoit, dans quelques décennies, un taux du nombre de porteurs d'une telle anomalie de l'hémoglobine atteignant 8 % de la population mondiale.

A l'heure actuelle, il existe plusieurs tests permettant le dépistage ou le diagnostic de la drépanocytose. Ces tests sont notamment basés sur les propriétés particulières de l'hémoglobine anormale, dite hémoglobine S. Ces propriétés sont par exemple, à l'état désoxygéné, une diminution de la solubilité et un changement de forme, appelé falciformation. En conditions d'hypoxie, l'hémoglobine des globules rouges subit en effet une gélification, et polymérise en fibres qui déforment le globule rouge en l'allongeant, ce qui lui confère un aspect de faucille.

Le caractère anormal drépanocytaire de l'hémoglobine peut ainsi être détecté par un test d'insolubilité, dit test ITANO, ou par un test de falciformation induite par hypoxie, tel que le test d'Emmel. Plus précisément, le test d'Emmel, bien connu de l'homme du métier, consiste à mettre un échantillon de sang en présence d'un agent réducteur, de sorte à déclencher le phénomène de falciformation des globules rouges drépanocytaires, et à observer au microscope l'effet de cet agent sur la forme des globules rouges. Ces tests nécessitent cependant un appareillage de laboratoire spécifique. En outre, ils ne sont pas automatisables.

Le document US 2012/0077218 décrit un procédé de diagnostic de la drépanocytose dérivé du test d'insolubilité d'Itano, qui consiste à soumettre un échantillon de sang à une étape de lyse cellulaire, en présence d'un agent réducteur dans un tampon phosphate, de sorte à provoquer la libération de l'hémoglobine S qui était contenue dans les globules rouges de l'échantillon, et sa désoxygénation et sa précipitation dans le tampon.

D'autres tests d'identification actuellement mis en œuvre reposent sur des techniques d'électrophorèse, de chromatographie en phase liquide haute performance (HPLC) ou des techniques relevant de la biologie moléculaire, notamment des techniques de détection de la disparition d'un site de restriction dans la séquence nucléotidique de l'hémoglobine, ou de réaction de polymérisation en chaîne (PCR). Outre le fait que ces tests nécessitent un appareillage spécifique et coûteux, ainsi qu'une expertise de l'opérateur, ils sont longs à mettre en œuvre.

La présente invention vise à remédier aux inconvénients des procédés de détection du caractère drépanocytaire d'un individu proposés par l'art antérieur, notamment à ceux exposés ci-avant, en proposant un procédé pour le dépistage de la drépanocytose à partir d'un échantillon de sang d'un individu, qui permette d'obtenir un résultat fiable tout en étant facile et rapide à mettre en œuvre. L'invention vise également à ce que les coûts associés à la mise en œuvre de ce procédé soient réduits.

Un objectif supplémentaire de l'invention est que ce procédé puisse être mis en œuvre, soit, d'une part, sans nécessiter d'appareillage complexe spécifique, ni expertise poussée de l'opérateur, soit, d'autre part, au moyen d'un dispositif automatisé.

A cet effet, la présente invention tire avantageusement parti d'une propriété particulière de l'hémoglobine S produite par l'individu drépanocytaire, à savoir la propriété de se polymériser, en conditions d'hypoxie, à l'intérieur du globule rouge. Comme expliqué ci-avant, en conditions d'hypoxie, la formation de polymères intracellulaires provoque une altération du cytosquelette de l'hémoglobine, à l'origine du phénomène de falciformation, ainsi que de la déshydratation et de la rigidification des érythrocytes. Ces modifications de déformabilité érythrocytaire contribuent à la crise vaso-occlusive, qui est la principale complication de la maladie.

Il a été découvert par les présents inventeurs qu'alors que les globules rouges normaux sont capables de se déformer, et de passer facilement dans des pores de taille bien inférieure à leur diamètre, notamment inférieure de quelques microns à ce diamètre, en revanche, les globules rouges drépanocytaires, ayant subi le phénomène de falciformation, en raison de leur forme en faucille et de leur caractéristique de rigidité particulières, ne sont pas, ou très peu, déformables, et sont incapables de passer dans des pores de même taille. Ainsi, il a été découvert par les présents inventeurs que la différence de déformabilité des globules rouges drépanocytaires, par rapport aux globules rouges normaux, rend possible la différenciation des échantillons de sang issus de patients drépanocytaires par rapport aux échantillons de sang issus de patients normaux, par filtration de tels échantillons à travers une membrane de taille de pores appropriée.

Il est ainsi proposé par la présente invention un procédé de dépistage de la drépanocytose chez un individu tel que défini dans la revendication 1, qui comprend les étapes successives suivantes, les étapes a/ et b/ pouvant être mises en œuvre successivement ou simultanément, le procédé ne comprenant pas d'étape de mise en contact de l'échantillon de sang avec un agent de lyse cellulaire :
a/ mise en contact d'un échantillon de sang, issu de l'individu dont on souhaite dépister s'il est atteint ou non de drépanocytose, avec un agent d'induction de la falciformation des globules rouges drépanocytaires, c'est-à-dire un agent apte à placer les globules rouges contenus dans l'échantillon de sang en conditions d'hypoxie, de sorte à provoquer la falciformation des globules rouges drépanocytaires,
b/ filtration de l'échantillon de sang, contenant des globules rouges, notamment le cas échéant des globules rouges ayant subi le phénomène de falciformation, à travers une membrane poreuse dont la taille de pores est déterminée de sorte à ce que ladite membrane soit apte à retenir les globules rouges ayant subi une falciformation, et à autoriser le passage des globules rouges n'ayant pas subi de falciformation,
c/ et détection, au cours et/ou à l'issue de l'étape b/ de filtration, de la présence éventuelle d'un résidu sur la membrane, une telle présence étant associée à un caractère atteint de l'individu pour la drépanocytose.

Un tel résidu éventuel se présente sous forme d'une gelée, formée d'agrégats de globules rouges en forme de faucille, de coloration rouge.

On entend dans la présente description, par l'expression « caractère atteint pour la drépanocytose », le fait que l'individu est du type homozygote SS, c'est-à-dire atteint par la maladie, ou hétérozygote AS, c'est-à-dire porteur de la maladie.

Le procédé selon l'invention est simple et rapide à mettre en œuvre. Il permet notamment un dépistage de la drépanocytose en quelques minutes, et qui plus est à faible coût. Les seuls consommables nécessaires à sa mise en œuvre sont l'agent d'induction de la falciformation et la membrane. Sa fiabilité a en outre été largement vérifiée par les présents inventeurs, et ce pour un grand nombre d'individus, aussi bien sains qu'atteints par la drépanocytose, par comparaison avec les résultats de tests biologiques de l'art antérieur réalisés pour les mêmes individus.

Le procédé selon l'invention est mis en œuvre à partir d'un échantillon de sang total, si bien qu'il ne nécessite pas d'équipements complexes pour le traitement préalable de l'échantillon de sang. En fonction des conditions de mise en œuvre du procédé, le seul prétraitement que peut avoir à subir cet échantillon est sa mise en présence avec un agent anticoagulant au moment de son prélèvement sur l'individu, par exemple en présence d'acide éthylènediaminetétraacétique (EDTA), de manière classique en elle-même pour les prélèvements sanguins pour analyses biologiques.

En particulier, le procédé selon l'invention ne comprend aucune étape de mise en contact de l'échantillon de sang avec un agent de lyse cellulaire, tel que la saponine. C'est bien un échantillon de sang contenant des globules rouges qui est soumis à l'étape b/ de filtration, et non une solution contenant de l'hémoglobine, qui pourrait être obtenue à partir de l'échantillon de sang par une étape de lyse cellulaire.

Selon la présente invention, cet échantillon de sang contenant des globules rouges peut être soumis à l'étape de filtration simultanément à l'étape de mise en contact avec l'agent d'induction de la falciformation des globules rouges drépanocytaires. Lorsqu'au contraire l'échantillon de sang est soumis à l'étape b/ de filtration après l'étape a/ de mise en contact avec l'agent d'induction de la falciformation des globules rouges drépanocytaires, cette étape b/ est préférentiellement réalisée directement après l'étape a/, c'est-à-dire qu'après la mise en œuvre de l'étape a/ de mise en contact avec l'agent d'induction de la falciformation, l'échantillon de sang, qui contient toujours des globules rouges, est directement soumis à l'étape b/ de filtration, sans subir d'étape intermédiaire de traitement, centrifugation, séparation de certains composants, etc.

Le procédé selon l'invention est applicable aussi bien chez l'adulte que chez le nouveau-né, pour un diagnostic précoce de la maladie, à partir d'un échantillon de sang prélevé du cordon ombilical. En particulier, même chez le nouveau-né, la différence d'aspect de la membrane à l'issue de l'étape de filtration, entre un échantillon de sang drépanocytaire et un échantillon de sang normal, est avantageusement visible à l'œil nu, malgré la faible quantité de globules rouges drépanocytaires présente dans le sang ombilical, y compris pour des échantillons de ce sang de faible volume. Le procédé selon l'invention peut de ce fait avantageusement s'inscrire dans un processus plus global non invasif pour le nouveau-né.

Un avantage supplémentaire du procédé selon l'invention est la possibilité d'archiver la membrane obtenue après l'étape de filtration, et sur laquelle un résidu formé de globules rouges drépanocytaires a été détecté, en vue de la mise en œuvre ultérieure de tests biologiques à partir de ces globules rouges drépanocytaires localisés sur la membrane, ou bloqués dans les pores de cette dernière. Par exemple, il peut alors être réalisé l'extraction de l'ADN de ces globules rouges, pour une analyse de cet ADN par des techniques de biologie moléculaire. Ceci s'avère notamment particulièrement avantageux lorsque le procédé de dépistage selon l'invention est mis en œuvre dans des pays en voie de développement, peu équipés en matériel d'analyse génétique. Après l'étape de filtration, les membranes obtenues pour les patients pour lesquels le procédé selon l'invention a permis d'établir un statut atteint par la maladie, peuvent être acheminées facilement vers des laboratoires de référence situés à distance du lieu du test, pour la mise en œuvre d'examens complémentaires.

L'archivage des membranes à l'issue du procédé selon l'invention permet en outre d'assurer la traçabilité des résultats.

L'agent d'induction de la falciformation des globules rouges drépanocytaires, apte à placer les globules rouges contenus dans l'échantillon de sang avec lequel il est mis en contact en conditions d'hypoxie, peut être de tout type classique en lui-même. Il entre dans les compétences de l'homme du métier d'identifier les agents répondant à une telle caractéristique, sur la base de ses connaissances générales et/ou de manière expérimentale. A cet effet, l'homme du métier pourra évaluer, pour un échantillon de sang mis en contact avec un agent d'induction de la falciformation candidat, la pression partielle en oxygène ou la saturation en oxygène, une pression partielle en oxygène faible et une saturation en oxygène diminuée étant des témoins gazométriques d'une hypoxie. Plus précisément, une pression partielle en oxygène inférieure ou égale à 20 mm Hg ou une saturation en oxygène comprise entre 30 et 60 % sont représentatives d'une hypoxie susceptible d'induire une falciformation chez un individu drépanocytaire.

Il entre également dans les compétences de l'homme du métier de déterminer la taille de pores de la membrane poreuse permettant de retenir les globules rouges ayant subi une falciformation et d'autoriser le passage des globules rouges n'ayant pas subi de falciformation. A cet effet, l'homme du métier pourra notamment s'appuyer sur le fait qu'un globule rouge normal présente un diamètre d'environ 8 µm, et que sa déformabilité lui permet de passer dans des capillaires de 2 à 3 µm, alors que les globules rouges drépanocytaires en sont incapables. La détermination de la taille de pores adéquates pourra par exemple être déterminée de manière empirique par l'homme du métier, à partir d'un échantillon de sang issu d'un individu sain vis-à-vis de la drépanocytose et d'un échantillon de sang d'un individu atteint de drépanocytose, par mise en conditions d'hypoxie de ces échantillons de sang, notamment selon la méthode d'Emmel, classique en elle-même, et test de filtration sur une membrane de taille de pores donnée, pour vérifier si cette taille de pores est ou non conforme à la présente invention, c'est-à-dire permet le passage des globules rouges normaux, en interdisant celui des globules rouges falciformes.

Dans des modes de mise en œuvre particuliers, le procédé selon l'invention répond en outre aux caractéristiques suivantes, mises en œuvre séparément ou en chacune de leurs combinaisons techniquement opérantes.

L'agent d'induction de la falciformation des globules rouges drépanocytaires est de préférence un agent réducteur qui, en consommant l'oxygène du milieu, de sorte que l'hémoglobine présente dans ce milieu se trouve en conditions d'hypoxie, provoque la réaction de falciformation des hématies drépanocytaires.

Dans des modes de mise en œuvre particuliers de l'invention, l'agent d'induction de la falciformation des globules rouges drépanocytaires est un sel de métabisulfite, par exemple le métabisulfite de sodium.

Dans des variantes de l'invention, la mise en contact de l'échantillon de sang avec l'agent d'induction de la falciformation des globules rouges drépanocytaires est réalisée par mélange de l'échantillon de sang avec une solution tampon de pH compris entre 6,8 et 7,4, contenant l'agent d'induction de la falciformation. Cette solution est notamment dépourvue d'agent de lyse cellulaire, tel que la saponine.

Cette solution peut contenir entre 1 et 10 %, de préférence entre 2 et 5 %, par exemple environ 5 %, en poids de l'agent d'induction de la falciformation, par rapport au volume total de la solution.

Une telle plage de concentration de l'agent d'induction de la falciformation dans la solution permet avantageusement de provoquer la falciformation des globules rouges drépanocytaires en un temps réduit.

Dans des modes de mise en œuvre particuliers de l'invention, la mise en contact de l'échantillon de sang avec l'agent d'induction de la falciformation est réalisée pendant une durée supérieure ou égale à 30 secondes, de préférence supérieure ou égale à 1 minute, et préférentiellement comprise entre 2 et 3 minutes. Une telle durée d'incubation de l'échantillon de sang avec l'agent d'induction de la falciformation des globules rouges drépanocytaires permet avantageusement d'obtenir un taux de falciformation suffisant pour que la différence d'aspect de la membrane à l'issue de l'étape de filtration soit décelable visuellement à l'œil nu.

Dans d'autres variantes de l'invention, la membrane utilisée pour la filtration est pré-imprégnée de l'agent d'induction de la falciformation, si bien que la mise en contact de l'échantillon de sang avec l'agent d'induction de la falciformation est réalisée au cours même de la filtration de l'échantillon de sang à travers la membrane. Le temps nécessaire à la mise en œuvre du procédé selon l'invention est ainsi d'autant plus court.

Dans d'autres variantes encore de l'invention, particulièrement avantageuses en termes de faisabilité et rapidité de mise en œuvre du procédé, la mise en contact de l'échantillon de sang avec l'agent d'induction de la falciformation est réalisée, préalablement à l'étape b/ de filtration, par dépôt de l'échantillon de sang sur un filtre poreux de taille de pores déterminée de sorte à être apte à autoriser le passage des globules rouges n'ayant pas subi de falciformation et le passage des globules rouges ayant subi ladite falciformation, et imprégné de l'agent d'induction de la falciformation. Ce filtre est disposé sur la membrane. On entend, par « disposé sur », le fait que le filtre est situé au-dessus de la membrane, dans le sens de l'écoulement de l'échantillon de sang, et de préférence en contact avec la membrane, de sorte à permettre le passage de l'échantillon de sang à travers le filtre jusqu'à la membrane par phénomène de capillarité.

Ce filtre peut par exemple présenter une taille de pores supérieure à 8 µm.

Un tel filtre peut notamment être du type absorbant, par exemple à base de fibres, notamment de fibres de coton, tels que les papiers de filtration commercialisés sous la marque Whatman®.

Ce filtre a été imprégné, préalablement à la mise en œuvre du procédé selon l'invention ou dans une étape préliminaire de celui-ci, par une solution contenant l'agent d'induction de la falciformation, par exemple à une concentration d'environ 25 % en poids, par rapport au volume total de la solution. Cette imprégnation peut notamment être réalisée par trempage du filtre dans la solution. Elle est préférentiellement suivie par une étape de séchage du filtre ainsi imprégné, par exemple à l'air libre et à température ambiante, ou sous léger chauffage.

Après séchage, ce filtre retient avantageusement une quantité suffisante d'agent d'induction de la falciformation pour induire la falciformation des globules rouges drépanocytaires contenus dans un échantillon de sang qui le traverse par effet de capillarité, avant de parvenir au contact de la membrane superposée sous le filtre.

L'induction de la falciformation se produit alors avantageusement en un temps très court, de l'ordre de quelques secondes.

Le procédé selon l'invention peut avantageusement être mis en œuvre à partir d'un volume très faible de sang, notamment compris entre 10 et 50 µl, de préférence compris entre 15 et 25 µl, par exemple compris entre 12 et 18 µl ou encore environ égal à 20 µl.

La membrane mise en œuvre pour l'étape de filtration peut être de tout type, la seule condition étant que les matériaux entrant dans sa constitution soient inertes vis-à-vis des composants de l'échantillon de sang. A titre d'exemple, la membrane utilisée est formée en polycarbonate.

Dans des modes de mise en œuvre particuliers de l'invention, la taille des pores de la membrane est comprise entre 2 et 8 µm, de préférence comprise entre 3 et 6 µm ou entre 6 et 7 µm. Une telle porosité autorise avantageusement le passage à travers la membrane des globules rouges issus du sang des individus normaux, ainsi que des leucocytes, tout en interdisant le passage des globules rouges drépanocytaires ayant subi le phénomène de falciformation.

La membrane peut en outre présenter tout taux de porosité.

La détection de la présence éventuelle d'un résidu sur la membrane de filtration peut être réalisée par observation visuelle de l'aspect de la surface de la membrane, en particulier à l'œil nu, ou par des moyens de détection optique du type à rayon infrarouge, à rayon laser, etc.

Dans des modes de mise en œuvre particuliers de l'invention, la détection de la présence éventuelle d'un résidu sur la membrane est réalisée par détection d'une éventuelle coloration rouge de la membrane, au cours et/ou à l'issue de l'étape de filtration.

Autrement, la détection de la présence éventuelle d'un résidu sur la membrane de filtration peut être réalisée par détection d'une surépaisseur de la membrane, due à l'amas de globules rouges drépanocytaires n'ayant pu traverser les pores de la membrane.

En outre, les globules rouges drépanocytaires ayant subi la falciformation, en restant sur la membrane au cours de la filtration, entrainent un colmatage de la membrane, par blocage des pores de cette dernière, et de ce fait une diminution du débit de la filtration. Il en résulte un temps de filtration plus long qu'en l'absence, dans l'échantillon, de globules rouges drépanocytaires. Cette différence de durée de la filtration, pour des conditions de filtration identiques, est de l'ordre de quelques dizaines de secondes, suffisante pour permettre de déterminer, par exemple en comparaison avec un individu normal témoin, si l'individu pour lequel un dépistage est réalisé est atteint ou non de drépanocytose.

Dans des modes de mise en œuvre particuliers de l'invention, la membrane est disposée sur un organe en matériau absorbant. On entend par là qu'un organe en matériau absorbant est situé au-dessous de la membrane, dans le sens de l'écoulement de l'échantillon de sang, et de préférence en contact avec la membrane, de sorte à permettre le passage de l'échantillon de sang à travers la membrane jusqu'à l'organe en matériau absorbant par phénomène de capillarité.

La détection de la présence éventuelle d'un résidu sur la membrane est réalisée par détection d'un éventuel changement de coloration de cet organe en matériau absorbant. L'absence d'un tel changement de coloration est représentative de la présence d'un résidu sur la membrane, et d'un individu atteint de drépanocytose. L'occurrence d'un tel changement de coloration, vers la couleur rouge, est quant à elle représentative de l'absence de résidu sur la membrane, et d'un individu sain vis-à-vis de la drépanocytose.

Cet organe en matériau absorbant peut par exemple être du type à base de fibres, tels que les papiers de filtration commercialisés sous la marque Whatman®.

Autrement, dans la configuration avantageuse de l'invention dans laquelle un filtre imprégné d'agent d'induction de la falciformation est disposé sur la membrane, la détection de la présence éventuelle d'un résidu sur la membrane peut être réalisée par détection d'une éventuelle coloration rouge de ce filtre, à l'issue de l'étape b/ de filtration. L'occurrence d'une telle coloration rouge est alors représentative de la présence d'un résidu sur la membrane, et d'un individu atteint de drépanocytose. L'absence d'une telle coloration rouge est quant à elle représentative de l'absence de résidu sur la membrane, et d'un individu sain vis-à-vis de la drépanocytose.

Dans des modes de mise en œuvre particulièrement avantageux de l'invention, la membrane est intercalée entre le filtre poreux et l'organe en matériau absorbant, de préférence de sorte à être en contact avec chacun d'eux, préalablement à la réalisation de l'étape a/ de mise en contact de l'échantillon de sang avec l'agent d'induction de la falciformation.

L'étape de filtration peut être réalisée soit par aspiration, soit par poussée, de l'échantillon de sang à travers la membrane, par des méthodes classiques en elles-mêmes pour l'homme du métier. Autrement, elle peut être réalisée par transfert par capillarité, la membrane de filtration étant alors par exemple superposée à un organe en matériau absorbant. Dans un tel mode de réalisation dans lequel la membrane de filtration est superposée à un organe en matériau absorbant, l'étape b/ de filtration peut également être assistée par aspiration ou poussée de l'échantillon de sang à travers la membrane.

La pression mise en œuvre pour l'étape de filtration est de préférence constante. Sa valeur est aisément déterminable par l'homme du métier, de manière théorique ou empirique, en fonction de la durée souhaitée pour l'étape de filtration, du volume d'échantillon à filtrer et des caractéristiques de la membrane mise en œuvre, notamment sa taille de pores et son taux de porosité. Préférentiellement, les conditions ci-avant du procédé selon l'invention sont choisies pour obtenir un temps de filtration court, par exemple d'environ deux minutes, voire moins.

Le procédé selon l'invention peut être mis en œuvre à partir de quelques microlitres à quelques millilitres de sang. Il peut notamment avantageusement être exécuté à partir d'environ 10 µl de sang, ce qui correspond sensiblement au volume d'une goutte de sang prélevée au doigt.

Des moyens pour la mise en œuvre d'un procédé selon l'invention peuvent être de différents types, chacun étant notamment particulièrement adapté à la configuration d'un type de territoire sur lequel le procédé selon l'invention est susceptible d'être mis en œuvre.

Un kit pour le dépistage de la drépanocytose chez un individu comporte un agent d'induction de la falciformation des globules rouges drépanocytaires, apte à placer les globules rouges contenus dans un échantillon de sang en condition d'hypoxie, et une membrane poreuse de taille de pores déterminée de sorte à être apte à retenir les globules rouges ayant subi ladite falciformation, et à autoriser le passage des globules rouges n'ayant pas subi de falciformation. Ce kit est dépourvu d'agent de lyse cellulaire.

L'agent d'induction de la falciformation et la membrane peuvent répondre à l'une ou plusieurs des caractéristiques énoncées ci-avant en référence au procédé de dépistage selon l'invention.

L'agent d'induction peut être imprégné sur la membrane.

Dans des variantes, le kit comporte une solution tampon de pH compris entre 6,8 et 7,4, contenant l'agent d'induction de la falciformation, et dépourvue d'agent de lyse cellulaire. Cette solution peut notamment répondre à l'une ou plusieurs des caractéristiques décrites ci-avant.

Par exemple, le kit peut comporter une solution tampon de pH compris entre 6,8 et 7,4, contenant du métabisulfite de sodium à 5 % en poids par volume, et une membrane de taille de pores de 3,5 µm, de 4,5 µm ou de 6,5 µm.

Dans des modes de réalisation différents, le kit comporte un filtre poreux de taille de pores déterminée de sorte à être apte à autoriser le passage des globules rouges n'ayant pas subi de falciformation et des globules rouges ayant subi ladite falciformation, et imprégné de l'agent d'induction de la falciformation.

Le kit peut en outre comprendre un organe en matériau absorbant, qui est de préférence apte à être superposé avec la membrane, au contact de cette dernière.

Ce filtre et cet organe en matériau absorbant peuvent répondre chacun à l'une ou plusieurs des caractéristiques décrites ci-avant les concernant en référence au procédé de dépistage de la drépanocytose selon l'invention. Ils peuvent être constitués dans le même matériau, ou dans des matériaux différents.

La membrane, le filtre et l'organe en matériau absorbants peuvent être présents dans le kit séparés les uns des autres. Autrement, ils peuvent y être présents sous forme d'un ensemble stratifié dont ils forment les couches constitutives qui sont de préférence plaquées les unes contre les autres, la membrane étant intercalée entre le filtre et l'organe en matériau absorbant. Cet ensemble peut être maintenu dans un support solide.

Dans des modes de réalisation particuliers, le kit comporte en outre un module de filtration, par exemple du type comportant, assemblés ou assemblables les uns aux autres :
- un réservoir pour l'échantillon à filtrer,
- un réceptacle de membrane, dans lequel la membrane de filtration peut être déjà positionnée, ou être destinée à être disposée,
- et un embout comportant un orifice pour l'évacuation du filtrat, ainsi, de préférence, que l'application d'une pression réduite provoquant l'aspiration du mélange contenu dans le réservoir à travers la membrane.

Ce module de filtration peut en outre comporter des moyens d'aspiration du liquide contenu dans le réservoir à travers la membrane, notamment un tube à pression interne réduite, tel qu'un tube Vacutainer®.

Le kit peut en outre comporter un réceptacle de collecte d'un échantillon de sang d'un individu, tel qu'un tube à pression interne réduite équipé d'un bouchon comportant un organe d'obturation transperçable, de type Vacutainer®, contenant le cas échéant un agent anticoagulant, par exemple de l'EDTA.

Le kit peut également comporter des instructions d'utilisation pour la mise en œuvre d'un procédé selon l'invention.

Préférentiellement, l'ensemble des constituants du kit sont de type jetable.

Un tel kit, facilement transportable, est particulièrement adapté pour une mise en œuvre dans les pays en voie de développement, par exemple dans des régions africaines, disposant de peu d'infrastructures d'analyse médicale.

Un dispositif pour la mise en œuvre d'un procédé selon l'invention comporte :
- des moyens automatisés de mise en contact de l'échantillon de sang issu de l'individu avec l'agent d'induction de la falciformation des globules rouges drépanocytaires,
- des moyens automatisés de filtration, à travers la membrane, de l'échantillon de sang contenant des globules rouges directement après l'étape de mise en contact de l'échantillon de sang avec l'agent d'induction de la falciformation des globules rouges drépanocytaires, c'est-à-dire sans étape intermédiaire autre que de déplacement, notamment sans étape intermédiaire de traitement, centrifugation, séparation de constituants, etc. Ces moyens automatisés de filtration peuvent être les mêmes que les moyens automatisés de mise en contact de l'échantillon de sang avec l'agent d'induction de la falciformation,
- et des moyens de détection optique de la présence éventuelle d'un résidu sur la membrane, au cours ou à l'issue de l'étape de filtration, par exemple des moyens de lecture de la membrane du type par rayon laser.

Ce dispositif peut en outre comporter un module automatisé de convoyage d'un réceptacle contenant l'échantillon de sang d'un des modules automatisés ci-avant à un autre, par exemple tel qu'un module communément désigné par l'expression « passeur d'échantillons », ainsi qu'un module de commande automatique d'un ou plusieurs des différents modules ci-avant pour la mise en œuvre d'une ou plusieurs des étapes d'un procédé de dépistage de la drépanocytose selon l'invention.

Un tel dispositif automatisé est particulièrement adapté pour une mise en œuvre dans les pays dits industrialisés, équipés de laboratoires d'analyse sophistiqués, pour le dépistage à haut débit.

Les caractéristiques et avantages de l'invention apparaîtront plus clairement à la lumière des exemples ci-après, fournis à simple titre illustratif et nullement limitatifs de l'invention, avec l'appui des figures 1 à 4, dans lesquelles :
- la figure 1 représente de manière schématique les constituants d'un kit pour le dépistage de la drépanocytose conformément à la présente invention, et illustre les différentes étapes d'un procédé de dépistage de la drépanocytose conforme à la présente invention, mis en œuvre au moyen de ce kit ;
- la figure 2 montre une photographie des membranes obtenues à l'issue de la mise en œuvre d'un procédé selon l'invention, à partir d'un échantillon de sang, (A) d'un individu normal, (B) d'un individu drépanocytaire, pour des taux de dilution de l'échantillon de sang dans une solution contenant un agent d'induction de la falciformation respectivement de 100, 50 et 10 µl d'échantillon de sang dans un volume total de 8,6 ml de cette solution ;

- la figure 3 montre des images obtenues par observation au microscope, avec un grossissement de 200 fois, des membranes de la figure 2, correspondant au volume de 10 µl d'échantillon de sang, (A) pour l'individu normal, (B) pour l'individu drépanocytaire ;
- et la figure 4 illustre de manière schématique les étapes d'un procédé de dépistage de la drépanocytose selon une variante de l'invention.

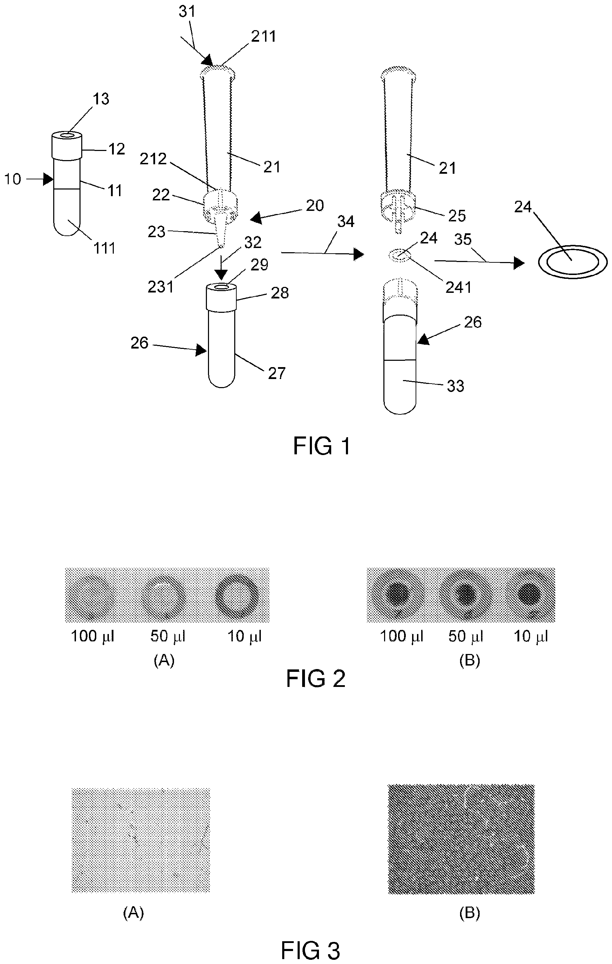

Un kit pour le dépistage de la drépanocytose selon l'invention est représenté de manière schématique sur la figure 1. Les dimensions relatives des différents constituants de ce kit ne sont pas représentatives de la réalité.

Ce kit comporte un premier tube 10 dit de prélèvement, comportant un corps 11 et un bouchon 12 comportant un organe d'obturation 13 pouvant être transpercé par une aiguille, connu sous le nom de septum. Un agent anticoagulant, en particulier de l'EDTA, est contenu dans le corps 11 du tube 10.

Le kit comporte en outre un module de filtration 20. Ce module de filtration peut notamment être tel que décrit dans les documents de brevet FR-A-2 952 069 ou FR-A-2 926 090 au nom de la présente déposante.

Schématiquement, le module de filtration 20 comporte les éléments suivants. Pour des raisons de clarté, certains de ces éléments sont représentés sur la figure 1 séparés les uns des autres, alors qu'ils sont dans la réalité initialement assemblés les uns aux autres.

Le module de filtration 20 comporte ainsi un réservoir 21 de réception de l'échantillon à filtrer, comportant, à une première extrémité 211, une ouverture pour l'alimentation en échantillon.

A une deuxième extrémité 212, notamment opposée à ladite première extrémité 211, le réservoir 21 est solidaire d'un organe 25 de réception d'une membrane de filtration 24. Cette membrane de filtration 24 est montée à cet effet dans un support de membrane rigide 241.

La membrane 24 est par exemple formée en polycarbonate. Elle présente par exemple une distribution des pores aléatoire, un diamètre de pores de 3,5 µm, et une densité de pores de 100 000 pores/cm².

Le module de filtration comporte en outre un embout creux 22, fixé de manière réversible autour de l'organe 25 de réception de la membrane, et comportant un support 23 pour une pointe de perçage d'un septum, percé d'un canal 231 débouchant à son extrémité, pour l'évacuation du filtrat et l'application de l'aspiration.

Le module de filtration comporte également un tube 26 à pression interne réduite, de type Vacutainer®, dit tube de collecte, comportant un corps 27 et un bouchon 28 à septum 29. La pression interne dans le tube de collecte 26 est par exemple initialement comprise entre 76 et 200 mm Hg.

Le kit comporte en outre une solution tampon à pH 7,2, dite solution réductrice, contenant pour 1 litre de solution, 8,41 g de tampon phosphate salin (PBS), 1,8 g d'EDTA, de l'eau distillée en quantité suffisante pour obtenir le volume total de 1000 ml. Le pH en est ajusté à la valeur souhaitée par de la soude (NaOH). L'agent réducteur, le métabisulfite de sodium, est ajouté à cette solution à une concentration de 5 % p/v.

Un procédé selon un mode de mise en œuvre particulier de l'invention est exécuté de la manière suivante, au moyen de ce kit.

Pour un individu à tester, un échantillon du sang est prélevé et recueilli dans le tube de prélèvement 10, selon le protocole standard de prise de sang dédiée à la prescription numéraire formule sanguine (NFS). Schématiquement, après avoir placé un garrot sur le bras, la veine radiale ou cubitale est ponctionnée à l'aide d'un dispositif sous vide, par exemple au moyen d'une aiguille biseautée reliée au tube de prélèvement 10. Un volume de sang 111, par exemple de 5 ml, est collecté dans le tube de prélèvement 10, et immédiatement mis au contact de l'EDTA.

Les échantillons sanguins sont de préférence utilisés dans les 24 h suivant le prélèvement.

Le volume souhaité de l'échantillon sanguin est ensuite prélevé du tube de prélèvement 10, et dilué dans la solution réductrice, pour atteindre le volume total souhaité. Les globules rouges drépanocytaires éventuellement contenus dans l'échantillon de sang subissent alors le phénomène de falciformation.

Après 1 à 2 min, la solution finale ainsi obtenue est placée dans le réservoir 21 du module de filtration 20, comme indiqué en 31 sur la figure 1. La pointe fixée sur le support de pointe 23 de l'embout 22 est ensuite insérée à travers le septum 29 du tube de collecte 26, comme indiqué en 32 sur la figure 1.

Du fait de la pression réduite dans le tube de collecte 26, il se produit une aspiration du contenu du réservoir 21, à travers la membrane 24. Le filtrat 33 traversant cette membrane 24 et recueilli dans le tube de collecte 26 comprend le sang contenant les globules rouges normaux et les leucocytes. Les globules rouges drépanocytaires rigides et en forme de faucille sont quant à eux retenus par la membrane 24.

A l'issue de la filtration, le module de filtration 20 est démonté, comme indiqué en 34 sur la figure 1, pour recueillir la membrane 24, comme indiqué en 35.

Cette membrane 24 est observée visuellement, pour en détecter une éventuelle coloration rouge et/ou détecter la présence éventuelle d'une surépaisseur sur sa surface. Une telle coloration et/ou une telle surépaisseur éventuelles sont indicatives d'un individu atteint de drépanocytose.

Un tel procédé selon l'invention a été mis en œuvre de la façon suivante, pour 16 patients atteints de drépanocytose et pour 16 patients normaux.

Le diagnostic biologique de la drépanocytose avait été établi préalablement soit par électrophorèse en présence d'ampholines (diagnostic dit de focussing de l'hémoglobine), soit par HPLC.

Des volumes respectivement de 100, 50 et 10 µl de sang de chacun des individus ont été dilués dans la solution réductrice, pour atteindre un volume total de 8,6 ml.

Pour chaque individu et chaque dilution, l'échantillon dilué ainsi obtenu a été placé dans le réservoir 21 d'un module de filtration 20, et soumis à filtration à travers la membrane 24.

La durée de la filtration a été comprise entre 1 et 2 min.

A l'issue de l'étape de filtration, chaque membrane a été récupérée et observée visuellement.

Pour les trois taux de dilution, un exemple des membranes obtenues pour un individu normal (A) et pour un individu drépanocytaire (B) est montré sur la figure 2. Ces résultats sont représentatifs de chaque catégorie d'individus testée.

On observe clairement une coloration des membranes issues de l'individu drépanocytaire, et ce pour tous les volumes initiaux d'échantillon de sang, y compris pour le volume de 10 µl. Cette coloration est rouge foncé dans la réalité. Aucune coloration n'est observée pour les membranes issues de l'individu sain.

Une observation au microscope des membranes obtenues pour un volume initial d'échantillon de sang de 10 µl a été réalisée. Les images obtenues sont montrées sur la figure 3. On y observe que la membrane utilisée pour la filtration de l'échantillon de sang normal ne comporte aucun globule rouge sur sa surface, alors que la membrane utilisée pour la filtration de l'échantillon de sang drépanocytaire comporte des agrégats de globules rouges sur sa surface.

Ces résultats sont représentatifs de l'ensemble des résultats obtenus, pour chacune des deux catégories d'individus testés. Pour l'ensemble des prélèvements réalisés, il a été observé un dépôt de globules rouges à la surface des membranes lorsque le sang provenait des patients atteints. Ce dépôt n'a jamais été observé lorsque le sang provenait d'individus sains. La falciformation induite par le métabisulfite de sodium a ainsi rendu les globules rouges drépanocytaires rigides et indéformables, incapables de pénétrer par déformabilité à travers les pores de la membrane, comme l'ont fait les globules rouges des individus normaux.

Ceci démontre clairement la fiabilité du procédé conforme à l'invention pour le dépistage de la drépanocytose. Même à partir d'un échantillon de sang d'un volume de 10 µl, ce procédé permet de discriminer les individus atteints de la maladie des individus sains.

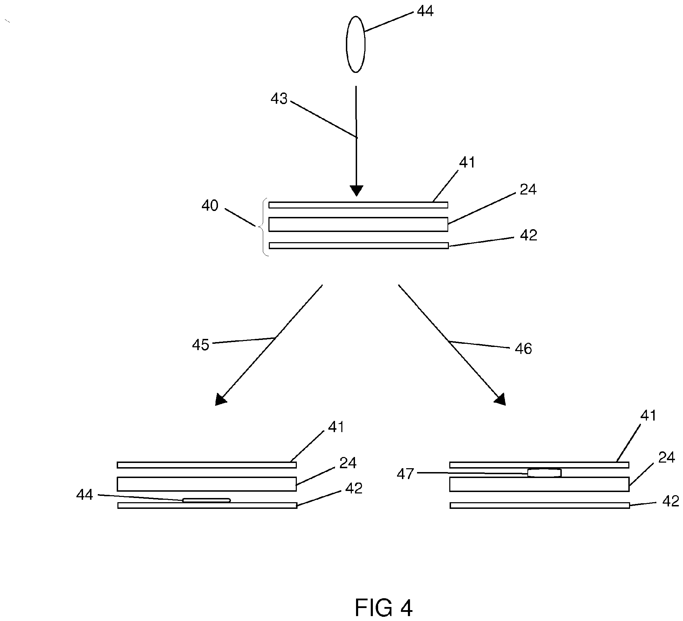

La figure 4 illustre un mode de mise en œuvre particulièrement avantageux de l'invention, à partir d'un kit comprenant un ensemble filtrant stratifié 40 comportant les couches constitutives successives suivantes : un filtre poreux 41, une membrane 24 et un organe en matériau absorbant 42. Sur cette figure, ces différentes couches sont représentées espacées les unes des autres pour des raisons de clarté. Elles sont dans la réalité en contact les unes avec les autres. Par ailleurs, les épaisseurs relatives de ces différentes couches ne sont en aucun cas représentatives de la réalité.

La membrane 24 répond aux caractéristiques décrites ci-avant.

Le filtre 41 et l'organe en matériau absorbant 42 peuvent être formés dans le même matériau, ou dans des matériaux différents.

Le filtre 41 présente une taille de pores suffisante pour autoriser le passage des globules rouges n'ayant pas subi de falciformation et des globules rouges ayant subi une falciformation.

Par exemple, le filtre 41 et l'organe 42 en matériau absorbant sont tous deux des papiers de filtration Whatman® n°40, à base de fibres de coton, d'épaisseur 22 µm, de grammage 95 g/m² et de rétention 8 µm.

Ce filtre 41 et cet organe en matériau absorbant 42 se présentent par exemple sous forme de disques, notamment de diamètre compris entre 8 mm et 2 cm, par exemple d'environ 90 mm.

Le filtre 41 a été préalablement imprégné par trempage dans une solution contenant l'agent d'induction de la falciformation, par exemple du métabisulfite de sodium, à une concentration de 25 % p/v, puis séché.

Au moyen d'un tel ensemble filtrant 40, un procédé de dépistage de la drépanocytose peut être mis en œuvre de la manière suivante.

Dans une première étape, illustrée en 43 sur la figure 4, un échantillon de sang 44 est déposé sur la surface supérieure du filtre 41. Le volume de cet échantillon de sang 44 est par exemple d'environ 15 µl, soit sensiblement l'équivalent d'une goutte de sang.

Cet échantillon traverse le filtre 41, par effet de capillarité. Au cours de cette phase, il se trouve au contact de l'agent d'induction de la falciformation. A ce contact, les globules rouges drépanocytaires éventuellement contenus dans l'échantillon de sang 44 subissent le phénomène de falciformation.

Selon que l'échantillon de sang provienne d'un individu atteint de drépanocytose ou d'un individu sain vis-à-vis de cette maladie, il se produit ensuite les évènements suivants.

Si l'individu est sain vis-à-vis de la drépanocytose, comme indiqué en 45 sur la figure 4, l'échantillon de sang 44, ne contenant pas de globules rouges ayant subi de falciformation, traverse la membrane 24, toujours par effet de capillarité, et est absorbé par l'organe en matériau absorbant 42. Ce dernier change de couleur, vers une coloration rouge. Le filtre 41 conserve quant à lui sa coloration initiale. Le dépistage d'une absence de caractère atteint pour la drépanocytose s'effectue ainsi par observation du filtre 41 et/ou de l'organe en matériau absorbant 42, le premier ayant conservé sa coloration initiale et le second s'étant coloré en rouge.

Si l'individu est atteint de drépanocytose, comme indiqué en 46 sur la figure 4, les globules rouges 47, contenus dans l'échantillon de sang 44 et ayant subi la falciformation, sont bloqués par la membrane 24. Une observation de l'organe en matériau absorbant 42 après quelques secondes montre que ce dernier a conservé sa coloration initiale, puisque les globules rouges ne sont jamais parvenus jusqu'à lui. Le filtre 41, quant à lui, est toujours au contact des globules rouges falciformes 47, dont une partie, contrairement à ce qui est représenté sur la figure 4, reste toujours coincée dans ses pores. Il présente par conséquent une coloration rouge. Ainsi, le dépistage d'un caractère atteint pour la drépanocytose s'effectue également par observation du filtre 41 et/ou de l'organe en matériau absorbant 42, le premier s'étant coloré en rouge et le second ayant conservé sa coloration initiale.

Ce test de dépistage a été avantageusement très simple et rapide à effectuer, qui plus est à partir d'un très faible volume de sang.

## Revendications

1. Procédé de dépistage de la drépanocytose chez un individu, **caractérisé en ce qu'**il comprend les étapes successives suivantes, les étapes a/ et b/ pouvant être mises en œuvre successivement ou simultanément, ledit procédé ne comprenant pas d'étape de mise en contact dudit échantillon de sang avec un agent de lyse cellulaire :
a/ mise en contact d'un échantillon de sang (44) issu dudit individu avec un agent d'induction de la falciformation des globules rouges drépanocytaires apte à placer les globules rouges contenus dans l'échantillon de sang en condition d'hypoxie,
b/ filtration dudit échantillon de sang (44) contenant des globules rouges à travers une membrane (24) poreuse de taille de pores déterminée de sorte à être apte à retenir les globules rouges ayant subi ladite falciformation (47), et autoriser le passage des globules rouges n'ayant pas subi de falciformation,
c/ et détection de la présence éventuelle d'un résidu (47) sur ladite membrane (24), au cours et/ou à l'issue de l'étape de filtration, ladite présence étant associée à un caractère atteint dudit individu pour la drépanocytose.

2. Procédé selon la revendication 1, selon lequel l'agent d'induction de la falciformation est un sel de métabisulfite.

3. Procédé selon l'une quelconque des revendications 1 à 2, selon lequel la porosité de la membrane (24) est comprise entre 2 et 8 µm.

4. Procédé selon l'une quelconque des revendications 1 à 3, selon lequel la mise en contact de l'échantillon de sang (44) avec l'agent d'induction de la falciformation est réalisée par mélange dudit échantillon de sang avec une solution tampon de pH compris entre 6,8 et 7,4 contenant ledit agent d'induction de la falciformation et dépourvue d'agent de lyse cellulaire.

5. Procédé selon la revendication 4, selon lequel ladite solution contient entre 1 et 10 % en poids dudit agent d'induction de la falciformation, par rapport au volume de ladite solution.

6. Procédé selon l'une quelconque des revendications 1 à 5, selon lequel la mise en contact de l'échantillon de sang (44) avec l'agent d'induction de la falciformation est réalisée pendant une durée supérieure ou égale à 30 secondes.

7. Procédé selon l'une quelconque des revendications 1 à 3, selon lequel la mise en contact de l'échantillon de sang (44) avec l'agent d'induction de la falciformation est réalisée, préalablement à l'étape b/ de filtration, par dépôt dudit échantillon de sang (44) sur un filtre poreux (41) de taille de pores déterminée de sorte à être apte à autoriser le passage des globules rouges n'ayant pas subi de falciformation et des globules rouges ayant subi ladite falciformation (47), et imprégné dudit agent d'induction de la falciformation, ledit filtre (41) étant disposé sur ladite membrane (24).

8. Procédé selon l'une quelconque des revendications 1 à 7, selon lequel la détection de la présence éventuelle d'un résidu (47) sur la membrane (24) est réalisée par détection d'une éventuelle coloration rouge de ladite membrane (24).

9. Procédé selon l'une quelconque des revendications 1 à 7, selon lequel la membrane (24) est disposée sur un organe en matériau absorbant (42), et la détection de la présence éventuelle d'un résidu (47) sur la membrane (24) est réalisée par détection d'un éventuel changement de coloration dudit organe en matériau absorbant (42), l'absence d'un tel changement de coloration étant représentative de la présence d'un résidu (47) sur la membrane (24).

## Patentansprüche

1. Verfahren zur Erkennung der Sichelzellkrankheit bei einer Person, **dadurch gekennzeichnet, dass** es die folgenden sukzessiven Schritte umfasst, wobei die Schritte a/ und b/ sukzessiv oder simultan durchgeführt werden können, wobei das Verfahren keinen Schritt des Inkontaktbringens der Blutprobe mit einem Zelllysemittel umfasst:
a/ Inkontaktbringen einer Blutprobe (44), die von der Person stammt, mit einem Mittel zur Induktion der Sichelzellbildung von roten Sichelzellenblutkörperchen, das dazu geeignet ist, die roten Blutkörperchen, die in der Blutprobe enthalten sind, in einen Hypoxiezustand zu versetzen,
b/ Filtrieren der Blutprobe (44), die rote Blutkörperchen enthält, durch eine poröse Membran (24) mit einer Porengröße, die so bestimmt ist, dass sie dazu geeignet ist, die roten Blutkörperchen, die der Sichelzellbildung unterzogen wurden (47), zurückzuhalten, und Zulassen des Hindurchtretens von roten Blutkörperchen, die nicht der Sichelzellbildung unterzogen wurden,
c/ und Nachweisen des eventuellen Vorhandenseins eines Rückstands (47) auf der Membran (24) im Verlauf und/oder zum Ende des Filtrationsschritts, wobei das Vorhandensein mit einem Charakteristikum der Person zur Entwicklung der Sichelzellkrankheit assoziiert ist.

2. Verfahren nach Anspruch 1, wobei das Mittel zur Induktion der Sichelzellbildung ein Metabisulfitsalz ist.

3. Verfahren nach einem der Ansprüche 1 bis 2, wobei die Porosität der Membran (24) zwischen 2 und 8 µm liegt.

4. Verfahren nach einem der Ansprüche 1 bis 3, wobei das Inkontaktbringen der Blutprobe (44) mit dem Mittel zur Induktion der Sichelzellbildung durch Mischen der Blutprobe mit einer Pufferlösung mit einem pH-Wert, der zwischen 6,8 und 7,4 liegt, die das Mittel zur Induktion der Sichelzellbildung enthält und die frei von einem Zelllysemittel ist, umgesetzt wird.

5. Verfahren nach Anspruch 4, wobei die Lösung zwischen 1 und 10 Gew.-% des Mittels zur Induktion der Sichelzellbildung enthält, bezogen auf das Volumen der Lösung.

6. Verfahren nach einem der Ansprüche 1 bis 5, wobei das Inkontaktbringen der Blutprobe (44) mit dem Mittel zur Induktion der Sichelzellbildung für einen Zeitraum umgesetzt wird, der länger als oder gleich 30 Sekunden ist.

7. Verfahren nach einem der Ansprüche 1 bis 3, wobei das Inkontaktbringen der Blutprobe (44) mit dem Mittel zur Induktion der Sichelzellbildung vor dem Schritt b/ der Filtration durch Abscheiden der Blutprobe (44) auf einem porösen Filter (41) mit einer Porengröße, die so bestimmt ist, dass sie dazu geeignet ist, das Hindurchtreten von roten Blutkörperchen, die nicht der Sichelzellbildung unterzogen wurden, und von roten Blutkörperchen, die der Sichelzellbildung unterzogen wurden (47), zulässt, und der mit dem Mittel zur Induktion der Sichelzellbildung imprägniert ist, umgesetzt wird, wobei der Filter (41) auf der Membran (24) angeordnet wird.

8. Verfahren nach einem der Ansprüche 1 bis 7, wobei das Nachweisen des eventuellen Vorhandenseins eines Rückstands (47) auf der Membran (24) durch Nachweisen einer eventuellen Rotfärbung der Membran (24) umgesetzt wird.

9. Verfahren nach einem der Ansprüche 1 bis 7, wobei die Membran (24) auf einem Element aus einem absorptionsfähigen Material (42) angeordnet wird und das Nachweisen des eventuellen Vorhandenseins eines Rückstands (47) auf der Membran (24) durch Nachweisen eines eventuellen Farbwechsels des Elements aus einem absorptionsfähigen Material (42) umgesetzt wird, wobei das Fehlen eines derartigen Farbwechsels das Vorhandensein eines Rückstands (47) auf der Membran (24) darstellt.

## Claims

1. A method of testing for sickle cell disease in an individual, **characterized in that** it comprises the following successive steps, it being possible for steps a/ and b/ to be carried out successively or simultaneously, said method comprising no step of bringing said blood sample into contact with a cell lysis agent:
a/ bringing a blood sample (44) from said individual into contact with an agent for inducing sickling of sickle red blood cells capable of placing the red blood cells contained in the blood sample in a hypoxic condition,
b/ filtering said blood sample (44) containing red blood cells through a porous membrane (24) of which the pore size is determined so as to be capable of retaining the red blood cells having undergone said sickling (47), and of allowing the red blood cells which have not undergone sickling to pass through,
c/ and detecting the possible presence of a residue (47) on said membrane (24), during and/or at the end of the filtering step, said presence being associated with the fact that the individual has a sickle cell disease characteristic.

2. The method as claimed in claim 1, wherein the agent for inducing sickling is a metabisulfite salt.

3. The method as claimed in either one of claims 1 and 2, wherein the porosity of the membrane (24) is between 2 and 8 µm.

4. The method as claimed in any one of claims 1 to 3, wherein the bringing of the blood sample (44) into contact with the agent for inducing sickling is carried out by mixing said blood sample with a buffer solution having a pH of between 6.8 and 7.4, containing said agent for inducing sickling and free of cell lysis agent.

5. The method as claimed in claim 4, wherein said solution contains between 1% and 10% by weight of said agent for inducing sickling, relative to the volume of said solution.

6. The method as claimed in any one of claims 1 to 5, wherein the bringing of the blood sample (44) into contact with the agent for inducing sickling is carried out for a period of greater than or equal to 30 seconds.

7. The method as claimed in any one of claims 1 to 3, wherein the bringing of the blood sample (44) into contact with the agent for inducing sickling is carried out, prior to the filtering step b/, by depositing said blood sample (44) on a porous filter (41) with a pore size that is determined so as to be capable of allowing red blood cells which have not undergone sickling and red blood cells having undergone said sickling (47) to pass through, and which is impregnated with said agent for inducing sickling, said filter (41) being placed on said membrane (24).

8. The method as claimed in any one of claims 1 to 7, wherein the detection of the possible presence of a residue (47) on the membrane (24) is carried out by detection of a possible red coloration of said membrane (24).

9. The method as claimed in any one of claims 1 to 7, wherein the membrane (24) is placed on a member made of absorbent material (42), and the detection of the possible presence of a residue (47) on the membrane (24) is carried out by detection of a possible change in coloration of said member made of absorbent material (42), the absence of such a change in coloration being representative of the presence of a residue (47) on the membrane (24).
